# EUROPÄISCHE PATENTANMELDUNG

(11) **EP 0 753 496 A2**
(43) Veröffentlichungstag der Anmeldung: **15.01.1997**
(21) Anmeldenummer: 96107033.1
(22) Anmeldetag: 04.05.1996
(51) Int. Cl.: C07B 35/02, C07C 47/02, C07C 5/05, C07C 45/62, C07C 253/30

(54) **Verfahren zur katalytischen Selektivhydrierung von mehrfach ungesättigten organischen Substanzen**

(30) Priorität: 08.07.1995 DE 19524971
(71) Anmelder: HÜLS AKTIENGESELLSCHAFT, 45764 Marl (DE)
(72) Erfinder: Büschken, Wilfried, Dr., 45721 Haltern (DE); Hummel, Jürgen, 45768 Marl (DE)

(57) **Zusammenfassung**

Die vorliegende Erfindung betrifft ein Verfahren zur katalytischen Selektivhydrierung von mehrfach ungesättigten organischen Substanzen, das dadurch gekennzeichnet ist, daß die Hydrierung in mehreren hintereinander geschalteten Schlaufen durchgeführt wird, wobei die mehrfach ungesättigten organischen Substanzen zusammen mit einem Teil des Hydrierproduktes des 1. Reaktors auf den Kopf des 1. Reaktors geleitet, der restliche Teil des Hydrierproduktes des 1. Reaktors zusammen mit einem Teil des Hydrierproduktes des 2. Reaktors auf den Kopf des 2. Reaktors geleitet und der restliche Teil des Hydrierproduktes des 2. Reaktors zusammen mit einem Teil des Hydrierproduktes des folgenden Reaktors auf den Kopf des folgenden Reaktors geleitet wird usw. und das Endprodukt aus dem restlichen Teil des Hydrierproduktes des letzten Reaktors gewonnen wird.

## Beschreibung

Die vorliegende Erfindung betrifft ein Verfahren zur katalytischen Selektivhydrierung von mehrfach ungesättigten organischen Substanzen.

Durch Verfahren zur katalytischen Selektivhydrierung, ausgehend von mehrfach ungesättigten organischen Substanzen, sind eine Reihe wichtiger organischer Verbindungen zugänglich. Ziel einer Selektivhydrierung ist es in der Regel, das gewünschte Hydrierprodukt in möglichst hoher Reinheit und mit möglichst hoher Ausbeute zu erhalten.

Die ausschließliche Herstellung einer Verbindung des Typs B durch Hydrierung einer ungesättigten Verbindung des Typs A wird dadurch erschwert, daß die Zielverbindung (Typ B) zu einer Verbindung des Typs C weiterreagieren kann.

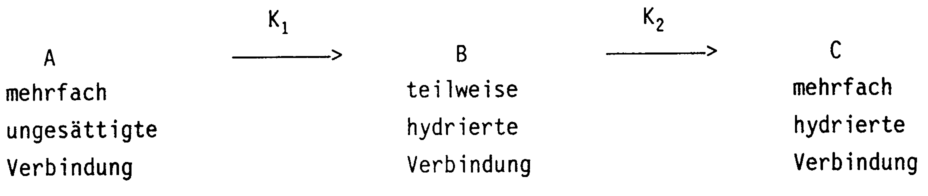

Für die kontinuierliche Selektivhydrierung in flüssiger Phase sind folgende Verfahrensweisen bekannt:
I) Die Hydierung wird einstufig in der sogenannten Schlaufe durchgeführt. Dadurch wird eine gute Wärmeführung und eine kurze Verweilzeit am Katalysator bewirkt.
II) Die Hydrierung wird zweistufig durchgeführt. Dabei wird in der ersten Stufe in der Schlaufe und in der zweiten Stufe im geraden Durchgang hydriert.

Der Nachteil der ersten Verfahrenweise besteht darin, daß kein vollständiger Umsatz von A erreicht wird. Dies bedeutet Ausbeuteverlust und, wenn B isoliert werden soll, einen erheblichen Aufwand für die Stofftrennung.

Bei der zweiten Verfahrensweise wird A vollständig hydriert. Nachteilig ist jedoch, daß auch Überhydrierung zu C eintritt. Dies bedeutet ebenfalls Ausbeuteverlust und Stofftrennung.

Es bestand daher die Aufgabe, ein Verfahren bereitzustellen, das es ermöglicht, mehrfach ungesättigte Verbindungen in wirtschaftlicher Weise katalytisch selektiv zu hydrieren.

Es wurde nun überraschend gefunden, daß ein praktisch vollständiger Umsatz bei gleichzeitig hoher Selektivität erreichbar ist, wenn man mehrere, d. h. mindestens zwei Schlaufen hintereinander geschaltet, betreibt.

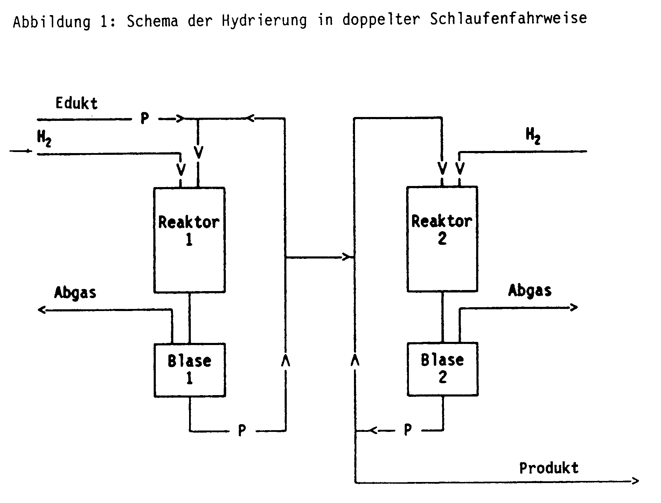

Abbildung 1 zeigt das Schema einer doppelten Schlaufenfahrweise. Dabei wird das Edukt A in einen Teil des Hydrieraustrags des ersten Reaktors eingespeist und dieses Gemisch auf den Kopf des ersten Reaktors geleitet. Aus der Hydriervorlage des ersten Reaktors wird standgeregelt Hydrierprodukt in die Schlaufe des zweiten Reaktors gefahren. Dieses Gemisch fließt auf den Kopf des zweiten Reaktors. Aus der Hydriervorlage des zweiten Reaktors wird standgeregelt das Endprodukt abgefahren. Beim vorliegenden Verfahren kann die Strömung in den Reaktoren turbulent oder laminar sein.

Das vorliegende Verfahren hat im Vergleich zur herkömmlichen, obengenannten Verfahrensweise II) (1. Stufe Schlaufe; 2. Stufe gerader Durchgang) folgende Vorteile:
1. Bei gleicher Katalysatormenge (Summe der Mengen in beiden Reaktoren) liegt die Produktionsmenge bis zu 90 % höher.
2. Die Selektivität (weniger überhydriertes Produkt) ist besser. Bei Umsätzen von 99,9 % und mehr werden Selektivitäten von über 99 % erreicht.

Gegenstand der vorliegenden Erfindung ist somit ein Verfahren zur katalytischen Selektivhydrierung von mehrfach ungesättigten organischen Substanzen, das dadurch gekennzeichnet ist, daß die Hydrierung in mehreren hintereinander geschalteten Schlaufen durchgeführt wird, wobei die mehrfach ungesättigten organischen Substanzen zusammen mit einem Teil des Hydrierproduktes des 1. Reaktors auf den Kopf des 1. Reaktors geleitet, der restliche Teil des Hydrierproduktes des 1. Reaktors zusammen mit einem Teil des Hydrierproduktes des 2. Reaktors auf den Kopf des 2. Reaktors geleitet und der restliche Teil des Hydrierproduktes des 2. Reaktors zusammen mit einem Teil des Hydrierproduktes des folgenden Reaktors auf den Kopf des folgenden Reaktors geleitet wird usw. und das Endprodukt aus dem restlichen Teil des Hydrierproduktes des letzten Reaktors gewonnen wird.

Vorzugsweise wird beim erfindungsgemäßen Verfahren die Hydrierung in zwei hintereinander geschalteten Schlaufen durchgeführt, wobei die mehrfach ungesättigten organischen Substanzen zusammen mit einem Teil des Hydrierproduktes des 1. Reaktors auf den Kopf des 1. Reaktors geleitet, der restliche Teil des Hydrierproduktes des 1. Reaktors zusammen mit einem Teil des Hydrierproduktes des 2. Reaktors auf den Kopf des 2. Reaktors geleitet und das Endprodukt aus dem restlichen Teil des Hydrierproduktes des 2. Reaktors gewonnen wird.

Das erfindungsgemäße Verfahren kann im allgemeinen in einem Temperaturbereich zwischen 0 und 200 °C und bei einem Druck von 1 bis 200 bar durchgeführt werden, wobei die Hydrierbedingungen vom Einsatzstoff bzw. dem gewünschten Hydrierprodukt abhängig sind. Dabei kann die Strömung im Reaktor laminar oder auch turbulent sein. Vorzugsweise wird beim erfindungsgemäßen Verfahren in allen Reaktoren in flüssiger Phase hydriert. Als Katalysatoren können beim erfindungsgemäßen Verfahren in der Regel marktgängige Hydrierkatalysatoren, beispielsweise 0,5 % Pd/Al₂O₃ (Fa. Engelhard), eingesetzt werden.

Als Edukte kommen im allgemeinen jene mehrfach ungesättigten Substanzen in Betracht, bei denen die teilweise hydrierte Verbindung B weniger leicht als die Ausgangsverbindung A hydriert wird.
Insbesondere ist das erfindungsgemäße Verfahren für die Herstellung folgender Verbindungsklassen vorteilhaft:
a) Herstellung von Olefinen aus Acetylenen
b) Herstellung von Olefinen mit mindestens einer isolierten Doppelbindung aus konjugierten Olefinen
c) Herstellung von gesättigten Ketonen aus ungesättigten Ketonen
d) Herstellung von gesättigten Nitrilen aus ungesättigten Nitrilen

Beim erfindungsgemäßen Verfahren können insbesondere Acetylene zu Olefinen selektiv hydriert werden und bei Olefinen mit konjugierten Doppelbindungen wird bevorzugt mindestens eine der in Konjugation stehenden Doppelbindungen unter Erhalt mindestens einer nicht in Konjugation stehenden Doppelbindung selektiv hydriert.

Bei organischen Substanzen mit mindestens einer Mehrfachbindung zwischen zwei Kohlenstoffatomen und mindestens einer Mehrfachbindung zwischen einem Kohlenstoffatom und einem Heteroatom kann eine oder mehrere Mehrfachbindungen zwischen zwei Kohlenstoffatomen beim erfindungsgemäßen Verfahren geeigneterweise auch unter Erhalt der Mehrfachbindungen zwischen einem Kohlenstoffatom und einem Heteroatom selektiv hydriert werden.

Ferner können beim erfindungsgemäßen Verfahren ungesättigte Ketone zu gesättigten Ketonen, ungesättigte Aldehyde zu gesättigten Aldehyden, beispielsweise 3-Propylhept-2-enal zu 3-Propylheptanal, aber auch ungesättigte Nitrile zu gesättigten Nitrilen sowie Aldolkondensationsprodukte von durch Hydroformylierung hergestellten Aldehyden zu gesättigten Aldehyden selektiv hydriert werden, wobei das erfindungsgemäße Verfahren aber nicht auf die zuvor genannten Beispiele beschränkt sein soll.

Durch das erfindungsgemäße Verfahren ist es möglich, eine Reihe ungesättigter Verbindungen durch eine katalytische Hydrierung mehrfach ungesättigter organischer Substanzen mit hoher Selektivität und hervorragender Ausbeute in einfacher und wirtschaftlicher Weise herzustellen.

## Patentansprüche

1. Verfahren zur katalytischen Selektivhydrierung von mehrfach ungesättigten organischen Substanzen,
dadurch gekennzeichnet,
daß die Hydrierung in mehreren hintereinander geschalteten Schlaufen durchgeführt wird, wobei die mehrfach ungesättigten organischen Substanzen zusammen mit einem Teil des Hydrierproduktes des 1. Reaktors auf den Kopf des 1. Reaktors geleitet, der restliche Teil des Hydrierproduktes des 1. Reaktors zusammen mit einem Teil des Hydrierproduktes des 2. Reaktors auf den Kopf des 2. Reaktors geleitet und der restliche Teil des Hydrierproduktes des 2. Reaktors zusammen mit einem Teil des Hydrierproduktes des folgenden Reaktors auf den Kopf des folgenden Reaktors geleitet wird usw. und das Endprodukt aus dem restlichen Teil des Hydrierproduktes des letzten Reaktors gewonnen wird.

2. Verfahren nach Anspruch 1,
dadurch gekennzeichnet,
daß die Hydrierung in zwei hintereinander geschalteten Schlaufen durchgeführt wird, wobei die mehrfach ungesättigten organischen Substanzen zusammen mit einem Teil des Hydrierproduktes des 1. Reaktors auf den Kopf des 1. Reaktors geleitet, der restliche Teil des Hydrierproduktes des 1. Reaktors zusammen mit einem Teil des Hydrierproduktes des 2. Reaktors auf den Kopf des 2. Reaktors geleitet und das Endprodukt aus dem restlichen Teil des Hydrierproduktes des 2. Reaktors gewonnen wird.

3. Verfahren nach den Ansprüchen 1 und 2,
dadurch gekennzeichnet,
daß in allen Reaktoren in flüssiger Phase hydriert wird.

4. Verfahren nach den Ansprüchen 1 bis 3,
dadurch gekennzeichnet,
daß die Strömung im Reaktor laminar ist.

5. Verfahren nach den Ansprüchen 1 bis 3,
dadurch gekennzeichnet,
daR die Strömung im Reaktor turbulent ist.

6. Verfahren nach den Ansprüchen 1 bis 5,
dadurch gekennzeichnet,
daR Acetylene zu Olefinen selektiv hydriert werden.

7. Verfahren nach den Ansprüchen 1 bis 5,
dadurch gekennzeichnet,
daR bei Olefinen mit konjugierten Doppelbindungen mindestens eine der in Konjugation stehenden Doppelbindungen unter Erhalt mindestens einer nicht in Konjugation stehenden Doppelbindung selektiv hydriert wird.

8. Verfahren nach den Ansprüchen 1 bis 5,
dadurch gekennzeichnet,
daß bei organischen Substanzen mit mindestens einer Mehrfachbindung zwischen zwei Kohlenstoffatomen und mindestens einer Mehrfachbindung zwischen einem Kohlenstoffatom und einem Heteroatom eine oder mehrere Mehrfachbindungen zwischen zwei Kohlenstoffatomen unter Erhalt der Mehrfachbindungen zwischen einem Kohlenstoffatom und einem Heteroatom selektiv hydriert werden.

9. Verfahren nach Anspruch 8,
dadurch gekennzeichnet,
daR ungesättigte Ketone zu gesättigten Ketonen selektiv hydriert werden.

10. Verfahren nach Anspruch 8,
dadurch gekennzeichnet,
daR ungesättigte Aldehyde zu gesättigten Aldehyden selektiv hydriert werden.

11. Verfahren nach Anspruch 10,
dadurch gekennzeichnet,
daß Aldolkondensationsprodukte von durch Hydroformylierung hergestellten Aldehyden zu gesättigten Aldehyden selektiv hydriert werden.

12. Verfahren nach Anspruch 11,
dadurch gekennzeichnet,
daß 3-Propylhept-2-enal zu 3-Propylheptanal selektiv hydriert wird.

13. Verfahren nach Anspruch 8,
dadurch gekennzeichnet,
daß ungesättigte Nitrile zu gesättigten Nitrilen selektiv hydriert werden.
